(19) Europäisches Patentamt
European Patent Office
Office européen des brevets

(11) EP 1 337 851 B1

(12) EUROPÄISCHE PATENTSCHRIFT

(45) Veröffentlichungstag und Bekanntmachung des Hinweises auf die Patenterteilung:
**20.07.2005 Patentblatt 2005/29**

(21) Anmeldenummer: **01996741.3**

(22) Anmeldetag: **14.11.2001**

(51) Int Cl.[7]: **G01N 33/58**, G01N 33/542

(86) Internationale Anmeldenummer:
**PCT/EP2001/013152**

(87) Internationale Veröffentlichungsnummer:
**WO 2002/041001 (23.05.2002 Gazette 2002/21)**

(54) **FARBSTOFFPAARE FÜR FLUORESZENZ-RESONANZ-ENERGIE-TRANSFER (FRET) MESSUNGEN**

DYE PAIR FOR FLUORESCENCE RESONANCE ENERGY TRANSFER (FRET) MEASUREMENTS

COUPLE DE COLORANTS POUR MESURES DE TRANSFERT D'ENERGIE DE FLUORESCENCE-RESONANCE

(84) Benannte Vertragsstaaten:
**AT BE CH CY DE DK ES FI FR GB GR IE IT LI LU MC NL PT SE TR**

(30) Priorität: **16.11.2000 EP 00124995**

(43) Veröffentlichungstag der Anmeldung:
**27.08.2003 Patentblatt 2003/35**

(73) Patentinhaber:
- **Roche Diagnostics GmbH**
  **68305 Mannheim (DE)**
  Benannte Vertragsstaaten:
  **DE**
- **F.HOFFMANN-LA ROCHE AG**
  **4070 Basel (CH)**
  Benannte Vertragsstaaten:
  **AT BE CH CY DK ES FI FR GB GR IE IT LI LU MC NL PT SE TR**

(72) Erfinder:
- **BELIK, Daniel**
  **82377 Penzberg (DE)**
- **JOSEL, Hans-Dieter**
  **82362 Weilheim (DE)**
- **HERRMANN, Rupert**
  **82362 Weilheim (DE)**
- **KOENIG, Bernhard**
  **82335 Berg (DE)**
- **MUELLER, Francis**
  **CH-4059 Basel (CH)**

(56) Entgegenhaltungen:
**WO-A-00/47693** **WO-A-98/43072**
**WO-A-99/51986**

- **BLOMBERG KAJ ET AL: "Terbium and rhodamine as labels in a homogeneous time-resolved fluorometric energy transfer assay of the beta subunit of human chorionic gonadotropin in serum." CLINICAL CHEMISTRY, Bd. 45, Nr. 6 PART 1, Juni 1999 (1999-06), Seiten 855-861, XP002167867 ISSN: 0009-9147 in der Anmeldung erwähnt**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft neue Fluoreszenz-Farbstoff-Systeme, speziell für Fluoreszenz-Resonanz-Energie-Transfer-Bestimmungen, zum Beispiel, in Kombination mit der zeitaufgelösten Messung der resultierenden Fluoreszenz. Die Erfindung betrifft zudem die Verwendung dieser Farbstoffe zur Markierung von Biomolekülen und deren Einsatz für die homogene Bestimmung von Wechselwirkungen zwischen Biomolekülen zum Beispiel beim Nachweis eines Analyten.

Zur Bestimmung von Biomolekülen werden sehr häufig Bindepartner, die an das gesuchte oder zu untersuchende Biomolekül spezifisch bindefähig sind, eingesetzt. Prinzipiell wird zwischen den heterogenen und den sogenannten homogenen Assays unterschieden, wobei Erstere dadurch charakterisiert sind, dass ein oder mehrere Waschschritte für die Durchführung des Tests notwendig sind.

In den sogenannten heterogenen Assays ist mindestens ein Biomolekül mit einer Markierungsgruppe versehen. Über die Messung dieser Markierungsgruppe wird letztlich die Konzentration des zu untersuchenden Analytmoleküls bestimmt. Natürlich ist diese Bestimmung nur dann sinnvoll möglich, wenn gebundene und ungebundene markierte Bindepartner vor Durchführung der Messung über einen geeigneten Waschschritt getrennt wurden.

In konventionellen homogenen Assays werden die Testbestimmungen so gewählt, dass über Trübungseffekte oder über Streulichteffekte messbare Signaländerungen in Abhängigkeit von der Konzentration des anwesenden Analytmoleküls erzeugt werden. Zur Verstärkung der generierten Signale kommen in solchen Assays häufig auch partikuläre Trägermaterialien zur Anwendung.

Erst in den letzten Jahren ist es möglich geworden, homogene Bestimmungen, d. h. Bestimmungen ohne dass zwangsläufig ein Waschschritt zwischengeschaltet sein müsste, auch unter Verwendung von Markierungsgruppen durchzuführen. Weiterentwicklungen auf dem Gebiet der homogenen Immunoassays beruhen allesamt auf der Wechselwirkung mindestens zweier Moleküle, die nur dann eintritt, wenn diese Moleküle sich in unmittelbarer Nähe zueinander befinden. Bekannt wurden homogene Assays, die auf den Prinzipien des "cloned enzyme donor immunoassays" = CEDIA (Microgenics Inc. USA), der "Fluoresenzpolarisation" = FPIA (Syva Co. USA) oder des "scintillation proximity assays" (Amershan, UK.) beruhen. An dieser Stelle sind aber auch insbesondere solche Methoden zu erwähnen, die auf dem Prinzip des Fluoreszenz-Resonanz-Energie-Transfers (FRET) beruhen. Für den Fluoreszenz Energie-Transfer sind immer mindestens zwei Farbstoffmoleküle notwendig. Ein erster Farbstoff, der als Energie-Donor und ein zweiter Farbstoff, der als Energie-Akzeptor fungiert. Die Energieübertragung zwischen Donor und Akzeptor erfolgt nicht radiativ, d. h., ohne Abgabe von Strahlung.

Die Effizienz des FRET ist stark abhängig von der Entfernung zwischen Donor- und Akzeptorfarbstoff. FRET erfolgt nur dann effizient, wenn Donor und Akzeptor sehr eng beieinander liegen.

Üblicherweise sind sowohl das Donormolekül, wie auch das Akzeptormolekül an jeweils einen Partner eines bioaffinen Bindepaares gebunden. Treten die Trägerbiomoleküle zueinander in Wechselwirkung und lagern sich z.B. zu einem Antigen-Antikörper-Komplex zusammen, so liegen auch Donor- und Akzeptormolekül sehr nahe beieinander und FRET ist möglich.

Die Energie Akzeptoren können entweder so gewählt werden, dass sie die vom Donor abgegebene Energie unterdrücken, man spricht hier von "Quenchern", oder die Fluoreszenz-Resonanz-Energie-Akzeptoren können ihrerseits selbst fluoreszente Energie abgeben, d. h. selbst fluoreszieren, man spricht von fluorophoren Gruppen oder kurz von "Fluorophoren". Aus dem Stand der Technik ist bekannt, dass metallische Komplexe sowohl als Fluoreszenz-Energie-Donoren, wie auch als Fluoreszenz-Energie-Akzeptoren in Frage kommen.

Wie oben erwähnt, sind auch fluorophore Gruppen als Akzeptoren in FRET-Systemen bekannt. Als solche Fluorophore werden vor allem Farbstoffe aus der Gruppe der Allophycocyanine (APCs) eingesetzt. Zu den bekannten Eigenschaften der APCs zählen, sowohl eine hohe Quantenausbeute, wie auch sehr gute Extinktionseigenschaften (z. B., EP 076 695).

Phycobiliproteine haben jedoch Nachteile, so ist, z. B., auf Grund des hohen Molekulargewichtes von über 100 000 d eine selektive Kopplung, d.h., über eine vorbestimmte Position im APC-Molekül, an ein Biomolekül nicht möglich. Diese Kopplung erfolgt meist chemisch und damit statistisch oder auch indirekt über Bindesysteme wie über das dem Fachmann bekannte Streptavidin/Biotin-System. Auch die Sensitivität, die untere Nachweisgrenze, dieser Systeme erscheint, z. B. beim Nachweis niedrig konzentrierter Analytmoleküle, als verbesserungswürdig.

Von Wallac, Oy, Turku, Finnland und Packard Instrument Company, Meriden, USA, sind kommerzielle Systeme erhältlich, die Lanthanid-Chelate als Donor-Label und Farbstoffe aus der Klasse der Phycobiliproteine, z. B., Allophycocyanin als Acceptor-Label verwenden. Die Lanthanid-Chelate besitzen eine Lumineszenz-Lebensdauer in einem Bereich bis zu einigen Millisekunden, d. h. entsprechend lange ist die Akzeptor-Emission zu beobachten. Die von Lanthanid-Chelaten abgegebenen Energie wird deshalb meist in einem Zeitfenster zwischen 400 - 600 Microsekunden gemessen. Dies bedeutet zwangsläufig auch, dass relativ lange Totzeiten existieren. Die Stabilität der Lanthanid-Chelate ist unter bestimmten TestBedingungen reduziert, so kann es beispielsweise durch Zugabe von Komplexbildnern, wie z. B., EDTA (Ethylen-di-Amino-tertra-Essigsäure) zu einer Umchelatisierung kommen.

US 5,998,146 beschreibt die Verwendung von Lanthanind-Chelat-Komplexen, insbesondere von Europium- und Terbium-Komplexen in Kombination mit Fluorophoren oder Quenchern. Dort sind auch die vorteilhaften Eigenschaften der langlebigen Lanthanid-Chelat-Komplexe unterstrichen.

Blomberg, et al., Clinical Chemistry 45(6) (1999) 855ff., beschreiben die Anwendung von Europium- oder Terbium-Komplexen als Donoren und eines Rhodamin-Farbstoffes als Akzeptor in neuen FRET-Paaren. Die Sensitivität des Nachweises von βhCG (β-Untereinheit des humanen chorionalen Gonadotropins) wird mit 0,43 μg/L angegeben. Die FRET-Assays basierend auf Europium- oder Terbium-Chelat-Komplexen führen somit zu keiner wesentlichen Verbesserung bezüglich der Sensitivität der Assays.

Die Verwendung von Ruthenium-Komplexen für die zeitaufgelöste Fluoreszenzmessung ist z. B. in EP-A2-439 036 beschrieben, dort wird Lumazin als Energie-Donor und der Ruthenium-Komplex als Energie-Akzeptor eingesetzt.

Joun et al., Analytical Biochemistry 232 (1995) 24-30 verwenden fluoreszierende Ruthenium-Komplexe als Energie-Donoren für homogene Bestimmungen, beruhend auf den FRET-Prinzip. Als Resonanz-Energie-Akzeptor wird der unter dem trivialen Namen "Reaktive Blue" bekannte Farbstoff eingesetzt. Reaktive Blue unterdrückt die vom Ruthenium-Komplex emitierte Fluoreszenz, so dass eine Quantifizierung anhand des unterdrückten Fluoreszenz-Signals, das ursprünglich vom Ruthenium-Komplex ausgegangen war, erfolgt.

In WO 00/47693 ist ebenfalls die Verwendung von Ruthenium-Chelat-Komplexen als Fluoreszenz-Energie Donoren in Kombination mit Quenchern beschrieben. Als Energie-Donor wurde der unter dem trivialen Namen "Fair Oaks Red ™" bekannte Ruthenium-Komplex eingesetzt. Dieser Farbstoff wurde an einen Antikörper gegen humanes Serumalbumin gekoppelt. Das Antigen, humanes Serumalbumin, wurde mit einem nichtlumineszierenden Farbstoff bekannt als "Light-Green Yellowish" markiert. Wie bei Joun, et al., (siehe oben) wurde die Analyt-Konzentration (humanes Serumalbumin) letztendlich über das Ausmaß an Signalunterdrückung bestimmt.

Für den Nachweis von Biomolekülen ist es in sehr vielen Fällen notwendig, dass auch geringe Mengen des zu untersuchenden Analytmoleküls nachgewiesen werden können. Um die Empfindlichkeit eines Meßsystems zu charakterisieren, wird häufig der Begriff der unteren Nachweisgrenze herangezogen. Je tiefer diese Nachweisgrenze liegt, desto sensitiver ist das Testsystem.

Bezüglich der Einfachheit der Kopplung von FRET-Farbstoffen an Biomoleküle und vor allem bezüglich der Sensitivität von Tests, die auf dem FRET-Prinzip beruhen, besteht jedoch noch erheblicher Verbesserungsbedarf. Empfindlichere homogene Testverfahren basierend auf FRET-Messungen wären in der Praxis breiter und vielseitiger einsetzbar und sind deshalb sehr erwünscht.

Aufgabe der vorliegenden Erfindung war es daher, neue FRET-Paare zu suchen und gegebenenfalls zu beschreiben, die die im Stand der Technik bekannten Nachteile, z. B. bezüglich der unteren Nachweisgrenze, überwinden können.

Es wurde überraschenderweise gefunden, dass metallische Chelat-Komplexe - basierend auf Metallionen der Gruppen VII und VIII der Übergangselemente - als Energie-Donoren in Kombination mit niedermolekularen Fluorophoren als Energie-Aktzeptoren mit großem Vorteil eingesetzt, zum Beispiel für sensitive Methoden zur Bestimmung der Wechselwirkung zwischen Biomolekülen beruhend auf dem FRET-Prinzip eingesetzt werden können.

<u>**Kurzbeschreibung der Erfindung:**</u>

Die Erfindung betrifft Methoden zur Bestimmung der Wechselwirkung zwischen mit Donor beziehungsweise Akzeptor markierten Biomolekülen beruhend auf dem Prinzip des Fluoreszenz-Energie-Transfer und der Messung der resultierenden Fluoreszenz, die dadurch gekennzeichnet sind, dass metallische Chelat-Komplexe - basierend auf Metallionen der Gruppen VII und VIII der Übergangselemente - als Energie-Donoren in Kombination mit niedermolekularen Fluorophoren mit einem Molekulargewicht zwischen 300 und 3000 d als Energie-Akzeptoren eingesetzt werden.

Es wurde überraschenderweise gefunden, dass niedermolekulare Fluorophore, speziell aus den Farbstoffklassen der Rhodamine, Xanthene, Cyanine und Oxazine hervorragend für FRET-Messungen, insbesondere in Kombination mit metallischen Chelat-Komplexen, basierend auf Metallionen der Gruppen VII und VIII der Übergangselemente, insbesondere Ruthenium-Chelat-Donoren geeignet sind, um verbesserte FRET-Assays aufzubauen.

Die erfindungsgemäßen FRET-Paare eigenen sich insbesondere für die zeitaufgelöste ("Time-resolved") Messung der resultierenden Energie (TR-FRET).

Die erfindungsgemäßen Farbstoffkombinationen eignen sich auch für Methoden zur Bestimmung der Wechselwirkung zwischen mit Donor beziehungsweise Akzeptor markierten Biomolekülen, welche auf dem Prinzip der Fluoreszenzmodulation beruhen.

Die neuen FRET-Paare können nunmehr in sehr vorteilhafter Weise eingesetzt werden, um Wechselwirkungen von Biomolekülen zu untersuchen, insbesondere dann, wenn die mit den FRET-Partnern markierten Biomoleküle sich räumlich zunächst sehr nahe sind und nach Wechselwirkung voneinander weiter entfernt vorliegen oder im umgekehrten Fall, wenn diese durch Wechselwirkungen, z. B. durch Ausbildung eines Komplexes zwischen den Partnern eines bioaffinen Bindepaares einander sehr nahe gebracht werden können.

Die Erfindung verbessert und erweitert die Möglichkeiten zum Nachweis einer Vielzahl von Analytmolekülen, speziell in sogenannten homogenen Testverfahren, und umfaßt deshalb auch Test-Kits zum Nachweis eines Analyten in einer Probe, welche mindestens ein mit einem Metall-Chelat-Komplex - basierend auf Metallionen der Gruppen VII und VIII der Übergangselemente insbesondere ein mit einem Ruthenium-Chelat-Komplex - markiertes Biomolekül, sowie ein mit einem niedermolekularen fluorophoren Akzeptor markiertes Biomolekül enthalten.

__Detaillierte Beschreibung der Erfindung:__

Die Erfindung betrifft in grundlegender Weise Methoden zur Bestimmung der Wechselwirkung zwischen mit Donor beziehungsweise Akzeptor markierten Biomolekülen beruhend auf dem Prinzip des Fluoreszenzenergietransfers und der Messung der resultierenden Fluoreszenz, dadurch gekennzeichnet, dass, metallische Chelat-Komplexe - basierend auf Metallionen der Gruppen VII und VIII der Übergangselemente - als Energie-Donoren in Kombination mit niedermolekularen Fluorophoren mit einem Molekulargewicht zwischen 300 und 3000 d als Energie-Akzeptoren eingesetzt werden.

Unter "Wechselwirkung" im Sinne der Erfindung sind über FRET-Messung erfassbare Abstandsveränderungen zwischen Biomolekülen zu verstehen. Zur Erfassung dieser Wechselwirkung ist es notwendig, dass sowohl ein FRET-Donor, wie auch ein FRET-Akzeptor an ein Biomolekül oder jeweils einen Partner eines Bindepaares gekoppelt sind und dass die Wechselwirkung zu einer Veränderung des Abstandes zwischen Donor und Akzeptor führt.

In einer bevorzugten Ausgestaltung ist jeweils ein Partner eines bioaffinen Bindepaares mit dem Donor bzw. mit dem Akzeptor markiert. Durch Ausbildung des Bindekomplexes zwischen den Partnern des bioaffinen Bindepaares werden Donor und Akzeptor einander stark angenähert, sodass FRET möglich ist.

Bekannte Beispiele für bioaffine Bindepaare sind insbesondere zueinander komplementäre Nukleinsäuresequenzen (DNA, RNA oder peptidische Nukleinsäuren), Liganden und Rezeptoren, Antigen oder Hapten und Antikörper, oder Lektine und Zucker. Unter geeigneten Bedingungen lagern sich die Partner dieser Bindepaare zu Komplexen zusammen.

Das Ausmaß an Komplexbildung wird bevorzugterweise dazu herangezogen, um die Konzentration eines Analytmoleküls zu bestimmen. Hierzu werden die Reaktionsbedingungen in der dem Fachmann bekannter Weise so gewählt, dass in Abhängigkeit von der Analytkonzentration, je nach Testformat, eine Signalzunahme oder - abnahme erfolgt.

Eine ebenfalls bevorzugte Ausgestaltung für eine Wechselwirkung im Sinne der Erfindung liegt vor, wenn Donor und Akzeptor ursprünglich unter Bedingungen vorliegen, die FRET ermöglichen, der FRET aber z. B. durch enzymatische Aktivität zwischen Donor- und Akzeptor-Kopplungsstelle unterbrochen wird.

Unter Fluoreszenz-Energie-Transfer versteht man den Übergang von Energie von einem Donor-Farbstoff auf einen Akzeptor-Farbstoff, wobei der Donor selbst möglichst wenig meßbare fluoreszente Energie emitiert. Bei diesem Prinzip wird ein Fluoreszenzfarbstoff-Donor, z. B. mit Licht einer geeigneten Wellenlänge angeregt. Aufgrund der räumlichen Nähe zu einem geeigneten zweiten Farbstoff, Akzeptor, findet daraufhin ein sogenannter nicht-radiativer, also strahlungsloser Energie-Transfer auf den Akzeptor statt (Van der Meer, et al., Resonance Energy Transfer VCH (1994)). Ist der zweite Farbstoff ein Fluorophor oder Luminophor so kann die Detektion des von diesem Molekül bei einer bestimmten Wellenlänge emittierten Lichtes sowohl zu einer qualitativen als auch zu einer quantitativen Bestimmung herangezogen werden.

In vielen Testsystemen, die auf diesem FRET-Prinzip beruhen, wird die als Donor fungierende luminophore Gruppe angeregt und durch Absorption eines Photons von einem Grundzustand in einen angeregten Zustand überführt. Falls sich das angeregte Donor-Molekül in ausreichender Nähe zu einem geeigneten Akzeptor-Molekül befindet, kann der angeregte Zustand vom Donor auf den Akzeptor übertragen werden. Dieser Energietransfer bewirkt, dass die Fluoreszenz oder Lumineszenz des Donors abnimmt und dass der Akzeptor für den Fall, dass er selbst lumineszierend ist, eine gesteigerte Lumineszenz zeigt. Falls es sich bei dem Akzeptor um einen Quencher handelt, so zeigt dieser selbstredend keine Fluoreszenz.

Die Effizienz der Energieübertragung ist sehr stark von der Entfernung zwischen dem Donor und dem Akzeptor-Molekül abhängig. Der Abstand wirkt sich auf die Signalabnahme in der 6. Potenz aus. Aufgrund dieser dramatischen Auswirkung des Abstands zwischen Donor und Akzeptor können FRET-Paare (man spricht auch von FRET-Systemen) benutzt werden, um zu untersuchen, wie viele Donor- und Akzeptor-Moleküle sich in unmittelbarer Nähe zueinander befinden. Diese Eigenschaft wird dann dazu ausgenutzt, um z. B. die Anwesenheit eines Analyten, beispielsweise durch In-Kontakt-Bringen mit einem spezifischen Partner, zu ermitteln. Eine Vielzahl von Anwendungsmöglichkeiten für FRET-Systeme sind aus dem Stand der Technik bekannt. Hierzu sei speziell auf WO 00/47693, EP 76 695; Hemmilä, Chemical Analysis 117, John Wiley&Sons, Inc., (1991)135-139; sowie auf Van der Meer, et al., Resonance Energy Transfer VCH (1994) supra, verwiesen.

Eine bevorzugte Ausgestaltung der FRET-Systeme sind solche Nachweismethoden, die sich zusätzlich der zeitverzögerten Messung des Signals aus einem FRET-System bedienen. Grundlegende Verrichtungen und Verfahren

zur Bestimmung der zeitaufgelösten FRET-Signale sind im Stand der Technik beschrieben. Das Prinzip der zeitaufgelösten FRET-Messungen beruht im Wesentlichen darauf, dass das Messfenster so gewählt wird, dass störende Hintergrundfluoreszenzen, die z. B. durch Störsubstanzen der Probe bedingt sein können, nicht mit erfasst werden, sondern nur die durch Energieübertragung erzeugte oder unterdrückte Fluoreszenz gemessen wird.

Die resultierende Fluoreszenz des TR-FRET Systems wird über entsprechend aufgebaute Messvorrichtungen ermittelt.

Solche "Time Resolved Detektionssysteme" benutzen z. B. gepulste Laser-Dioden, Light-Emitting-Diodes (LEDs) oder gepulste Farbstofflaser als Anregungslichtquelle. Die Messung erfolgt nach entsprechender Zeitverzögerung also nach dem Abklingen der störenden Hintergrundsignale. Der prinzipielle Aufbau einer solchen Messeinrichtung ist in Abbildung 1 dargestellt. Kommerziell erhältliche Messsysteme, z. B. basierend auf Xenonblitzlampen, wie z. B. Victor™ von Wallac Oy, sind für die sensitive Bestimmung der zeitverzögerten Fluoreszenz im Bereich von wenigen μs, wie sie für die erfindungsgemäßen FRET-Paare notwendig ist, nicht geeignet, sondern nur für FRET-Systeme ab 10μs Lebenszeit.

Die Detektion kann bevorzugt auch mit der Phasenmodulationstechnik erfolgen. Hierbei wird die Intensität des Anregungslichtes mit hoher Frequenz moduliert, die Intensität der Emission dadurch ebenfalls. Die Fluoreszenzemission ist aufgrund der Lebensdauer phasenverschoben und demoduliert. Auf einschlägige Informationen zu entsprechenden Systemen wird an dieser Stelle ausdrücklich hingewiesen (WO 00/47693; French, et al., SPIE BiOS in Proc. SPIE v 3259 (1998) 209-218 und French, et al., SPIE BiOS in Proc. SPIE v 3603 (1999) 272-280). Der Fachmann findet dort die notwendigen Angaben, um die erfindungsgemäßen Farbstoffkombinationen auch in solchen Fluoreszenzmodulations-Systemen mit Erfolg einzusetzen. Im folgenden wird im Wesentlichen nur auf TR-FRET Bezug genommen, dennoch ist für den Fachmann offenkundig, dass er sich auch der Phasenmodulationstechnik bedienen kann, um die erfindungsgemäßen FRET-Paare zu messen.

FRET-Systeme basierend auf metallischen Komplexen als Energie-Donoren und Farbstoffen aus der Klasse der Phycobiliproteine als Energie-Akzeptoren sind im Stand der Technik bekannt (EP 76 695; Hemmilä, Chemical Analysis 117, John Wiley&Sons, Inc., (1991)135-139. Etablierte kommerzielle Systeme (z. B. von Wallae, OY, oder Cis Bio-Packard) verwenden ein FRET-Paar, das aus einem Lanthanid-Chelat als metallischem Komplex einerseits und einen Phycobiliprotein andererseits besteht.

Die vorteilhaften Eigenschaften der Lanthanid-Chelat-Komplexe, insbesondere von Europium- oder Terbium-Komplexen sind bekannt und können sowohl in Kombination mit Quenchern, wie auch in Kombination mit Fluorophoren genutzt werden. Die Kombination solcher Lanthanid-Chelat-Komplexe mit niedermolekularen Fluorophoren scheint keine wesentliche Verbesserung der Sensitivität zu erbringen (US 5,998,146 und Blomberg, et al., supra).

Ruthenium-Komplexe per se werden als Fluorophore bzw. Luminophore speziell in der Elektro-Chemie-Lumineszenz eingesetzt. Bevorzugte Ruthenium-Chelat-Komplexe sind z. B. aus EP 178 450 und EP 772 616 bekannt. Dort sind auch bevorzugte Kopplungsmethoden dieser Komplexe an Biomoleküle beschrieben. Die Verwendung als Energie-Donoren in FRET-Systemen ist dort nicht diskutiert.

Allophycocyanine weisen herausragende Eigenschaften, z. B., einen außergewöhnlich hohen Extinktionskoeffizienten (rund 700000 L/M cm) und ebenso einen außerordentlich hohen Emissionskoeffizienten auf. Dies sind ideale Voraussetzungen bezüglich ihrer Eignung als fluorophore Akzeptoren in FRET-Systemen. Zudem sind diese Farbstoffe als gut wasserlöslich und stabil bekannt.

Der Begriff niedermolekulare Fluorophore umfaßt fluorophore Farbstoffe mit einem Molekulargewicht zwischen 300 und 3000 d. Solche niedermolekularen fluorophoren Gruppen, wie z. B. Xanthene, Cyanine, Rhodamine und Oxazine, weisen im Vergleich zu den APCs bezüglich wichtiger Charakteristika erhebliche Nachteile auf. So sind z. B. die Extinktionskoeffizienten deutlich niedriger und liegen im Bereich von ca. 100 000 L/M cm. Ebenso ist bekannt, dass unspezifische Bindungen aufgrund der hydrophoben Eigenschaften dieser Chromophore als potentielle Nachteile für diese Farbstoffe als Akzeptoren in FRET-Systemen anzuführen sind.

Überraschenderweise wurde nun gefunden, dass die erfindungsgemäßen FRET-Paare, bestehend aus einem Metall-Chelat-Komplex mit Metallionen aus der VII. und VIII. Gruppe der Übergangselemente, bevorzugt Rhenium, Osmium, Iridium oder Ruthenium, besonders bevorzugt Ruthenium einerseits und einem niedermolekularen Fluorophor andererseits, große Vorteile, speziell bezüglich der Sensitivität, in FRET-Messungen aufweisen.

Es hat sich gezeigt, dass die in dieser Erfindung beschriebenen Farbstoffkombinationen erstaunlicherweise zu sehr empfindlichen Testsystemen und sogar zur Verbesserung der unteren Nachweisgrenze, z. B., für Messverfahren beruhend auf dem Prinzip der zeitverzögerten Messung in FRET-Systemen führen.

Es wurde weiter gefunden, dass diejenigen Farbstoffe aus den oben genannten Farbstoffklassen, die ein Absorptionsmaximum bei einer Wellenlänge zwischen 600 nm und 750 nm haben, besonders geeignet sind. Eine bevorzugte Ausgestaltung der hier anhängigen Erfindung stellt demnach eine Methode zur Bestimmung der Wechselwirkung zwischen mit Donor beziehungsweise Akzeptor markierten Biomolekülen beruhend auf dem Prinzip des Fluoreszenz-Engerie-Transfers und z. B. der zeitverzögerten Messung der resultierenden Fluoreszenz dar, die gekennzeichnet ist durch die kombinierte Verwendung von Metall-Chelat-Komplexen, wie oben beschreiben und von niedermolekularen Fluorophoren mit einem Absorptionsmaximum zwischen 600 nm und 750 nm.

Wird ein Ruthenium-Komplex, wie z. B. in EP 178 450 oder EP 772 616 beschrieben, als Donor in einem FRET-System eingesetzt, so hat ein besonders geeignetes Akzeptor-Molekül ein Absorptionsmaximun bei Wellenlängen zwischen 600 nm und 750 nm und ganz besonders bevorzugt im Wellenlängenbereich zwischen 630 nm und 700 nm.

In einer besonders bevorzugten Ausgestaltung der Erfindung ist das niedermolekulare fluorophore Molekül weiter dadurch gekennzeichnet, dass es ein Molekulargewicht von von kleiner 2000 d oder bevorzugt von weniger als 1500 d oder besonders bevorzugt von weniger als 1000 d aufweist. Das Molekulargewicht von z. B. 1000 d bezieht sich dabei auf die Farbstoffkomponente als solche, also z. B. nicht auf zusätzliche Linkerstrukturen oder andere Kopplungsprodukte. Bevorzugterweise hat das niedermolekulare Fluorophor mindestens ein Molekulargewicht von 300 d, besonders bevorzugt von mindestens 350 d.

Als besonders geeignet haben sich Farbstoffe aus den Substanzklassen der Xanthene, Cyanine, Rhodamine und Oxazine erwiesen. In einer besonders bevorzugten Ausgestaltung der Erfindung, wird deshalb der niedermolekulare fluorophore Farbstoff aus einer Gruppe, die Xanthene, Cyanine, Rhodamine und Oxazine umfaßt, ausgewählt.

Farbstoffe aus der Gruppe Rhodamine sind in EP 567 622 detailliert beschrieben. Dort ist ebenfalls ausgeführt, welche Maßnahmen ergriffen werden können, um RhodaminFarbstoffe zu erzielen, deren Absorptionsmaximum in Richtung des längerwelligen Lichtes verschoben ist. Fluorophore aus der Klasse der Rhodamine der folgenden allgemeinen Formel (Formel I) sind als niedermolekulare Fluorophore besonders bevorzugt.

Formel I: Rhodamine

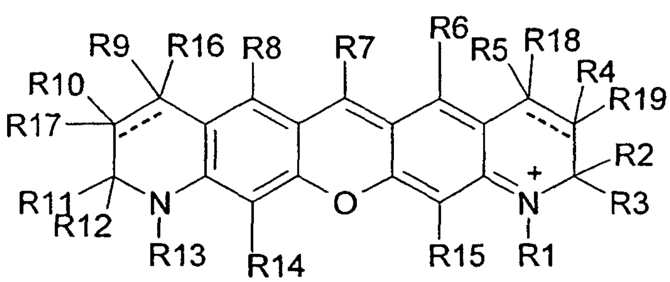

worin R1 und R13 gleich oder verschieden sind und bedeuten: Wasserstoff, Alkyl mit 1 bis 20 Kohlenstoffatomen, Polyoxyhydrocarbyl-Einheiten, Phenyl, Phenylalkyl mit 1 bis 3 Kohlenstoffatomen in der Alkylkette, worin die Alkyl- oder/und Phenylreste durch eine oder mehrere Hydroxy-, Halogen-, Sulfo-, Carboxy- oder Alkoxycarbonylgruppen, worin Alkoxy 1 bis 4 Kohlenstoffatome besitzen kann, substituiert sein können; R7 eine durch mindestens ein Halogen substituierte Alkylgruppe mit 1 bis 20, vorzugsweise 1 bis 7 Kohlenstoffatomen oder eine Phenylgruppe, die durch eine in o-Stellung zu dem an das pentacyclische Ringsystem gebundene Kohlenstoffatom befindliche Carboxy- oder Alkoxycarbonylgruppe und mit mindestens 1 Halogen substituiert ist, und worin Alkoxy 1 bis 4 Kohlenstoffatome besitzen kann, oder eine Carboxyalkylgruppe oder eine Carboxymethylenoxy-alkyloxygruppe bedeutet;

die beiden strichliniert eingezeichneten Bindungen bedeuten, dass die beiden durch die strichlinierte Bindung verbundenen Kohlenstoffatome durch eine Einfach- oder Doppelbindung miteinander verbunden sein können; wobei R14, R15 und die übrigen, nicht mit spezifischen Symbolen bezeichneten Positionen des pentacyclischen Grundkörpers durch Alkylgruppen mit 1 bis 20 Kohlenstoffatomen substituiert sein können; wobei X- ein Gegenion ist; und wobei zumindest einer der Reste R1, R7 oder/und R13 an ein Biomolekül gekoppelt ist.

Ganz besonders bevorzugt sind solche Rhodamine, bei welchen der Rest R7 eine stark elektronenziehende Gruppe ist. Solche elektronenziehenden Gruppen an R 7 sind bevorzugterweise Polyhalogencarboxyphenyl- und Perfluoralkylreste. Besonders bevorzugt sind Tetrachlorcarboxyphenylreste und Polyhalogenphenylreste in Position R 7. Diese zeigen eine besonderes gute Stabilität in weiten pH Bereichen. Die Feinabstimmung der Wellenlänge in den Rhodaminfarbstoffen kann durch Einführung von Doppelbindungen und zusätzlich durch Methyl Substituenten an den Resten R 2, 3, 11, 12, 5 und/oder 9 erfolgen. Die Anknüpfung an Biomoleküle erfolgt bevorzugt über die Reste R1 oder R 13. Zusätzlich lassen sich auch die Linkerlängen hinsichtlich der Testperformance optimieren.

Auch die Hydrophilie solcher pentacyclischen Rhodaminfarbstoffe ist durch Substitution mit entsprechenden hydrophilen Gruppen in weiten Bereichen modifizierbar. Bevorzugterweise werden Sulfonsäuregruppen verwendet, die grundsätzlich an beliebigen Positionen eingeführt werden können. Erfolgt die Einführung der Sulfonsäuregruppe

in einer der Positionen R 1 und R 13 so ist die andere Position bevorzugt mit Carboxyalkyl substituiert.

In einer weiteren bevorzugten Ausgestaltung werden Oxazine der folgenden allgemeinen Formel als Fluoreszenzresonanz-Energie-Akzeptoren eingesetzt.

Formel II: Oxazine

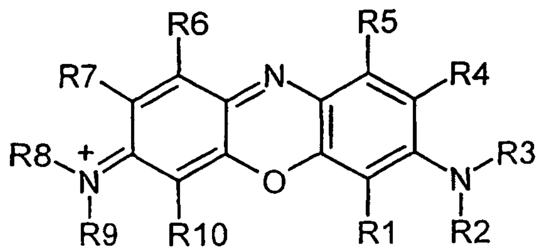

worin R1, R4, R5, R6, R7, R10 Wasserstoff, Alkyl, Hydroxy, Halogen, Carboxyl, Sulfonyl oder Amino bedeutet und R2, R3, Wasserstoff, Alkyl, Alkoxy, Polyoxyhydroxycarbonyleinheiten, Phenyl, Phenylalkyl bedeutet, die durch Hydroxy, Sulfonyl, Carboxy, Amino, Alkoxycarbonyl substituiert sein können, wobei R2 mit R1 oder R3 mit R4 eine gesättigte oder ungesättigte C4- oder C5-Brücke bilden kann und

R8, R9, Wasserstoff, Alkyl, Alkoxy, Polyoxyhydroxycarbonyleinheiten, Phenyl, Phenylalkyl bedeutet, die durch Hydroxy, Sulfonyl, Carboxy, Amino, Alkoxycarbonyl substituiert sein können, wobei R2 mit R1 oder R3 mit R4 eine gesättigte oder ungesättigte C4- oder C5-Brücke bilden kann und

wobei mindestens eine der Reste R2, R3, R8 oder R9 einen nicht-brückebildenden Rest darstellt, der an ein Biomolekül gekuppelt ist und wobei mindestens einer der Reste R2, R3, R8 und R9 einen brückebildenden Rest darstellt, der gegebenenfalls durch Alkyl substituiert sein kann, darstellt

Oxazine und deren Kopplung an Biomoleküle sind in EP 747 447 offenbart. Auf die dortige Beschreibung zu den Oxazin-Farbstoffen und die bevorzugten Ausgestaltungen wird hiermit Bezug genommen. Oxazinfarbstoffe, bei welchen R3, R4 und/oder R7, R8 eine Ringstruktur ausbilden sind besonders bevorzugt, da hierdurch die Quantenausbeute wesentlich verbessert wird. Das "Feintuning" der Absorptionswellenlänge und der Hydrophilie kann wie oben für Rhodamine beschrieben erfolgen.

Weiter bevorzugte Farbstoffe werden aus den Klassen der Cyanine (siehe Mujumdar, et al., Bioconjugate Chem. 7, 1996, 356-362) oder der Xanthene (EP 1 054 039) ausgewählt werden.

Zur Bestimmung der Sensitivität, die von einem zu untersuchenden FRET-Paar erreicht werden kann, sind, je nach Test, Analyt und Binde- oder Nachweisreagenz, sehr viele Testkonfigurationen denkbar und möglich. Solchen Systemen mangelt es jedoch offensichtlich an der Vergleichbarkeit zueinander und an der Übertragbarkeit auf andere Systeme.

Zweckmäßigerweise wird als Standardsystem zur Bestimmung der unteren Nachweisgrenze (UNG) das Biotin-Streptavidin-System herangezogen. Das niedermolekulare Biotin wird von Streptavidin sehr fest gebunden. Dieses hochaffine Bindepaar ermöglicht eine reproduzierbare vergleichende Bestimmung der Sensitivitäten, wie sie mit unterschiedlichen FRET-Paaren erreicht werden können.

Es ist bevorzugt, dass das Biotin-Streptavidin-System eingesetzt wird, um die untere Nachweisgrenze für ein zu untersuchendes FRET-Paar zu ermitteln. Dazu wird der FRET-Energie-Donor-Komplex an Streptavidin, wie in Beispiel 1b) beschrieben, angebunden. Der niedermolekulare Akzeptor-Farbstoff wird mit Diamino-dioxa-octan (DADOO) als Linker an Biotin gekoppelt (vgl. Beispiele 1d) und 1e)). Die Bestimmung der Sensitivität erfolgt bevorzugt wie in Beispiel 3 beschrieben.

Die erfindungsgemäßen FRET-Paare können mit der oben beschriebenen Vorgehensweise so gewählt werden, dass die untere Nachweisgrenze verbessert wird. Bevorzugt sind solche FRET-Paare, die aus Metallionen der VII. und VIII. Gruppe der Übergangselemente als Donoren und niedermolekularen Akzeptoren bestehen, die unter den oben definierten Bedingungen eine untere Nachweisgrenze von □ $3{,}0 \times 10^{-13}$M aufweisen. Bevorzugte FRET-Paare weisen eine untere Nachweisgrenze von □$2 \times 10^{-13}$M, besonders bevorzugte FRET-Paare eine untere Nachweisgrenze von □ $1 \times 10^{-13}$M auf.

Die Erfindung betrifft somit ebenfalls ein verbessertes Verfahren zur Bestimmung der Wechselwirkung zwischen mit Donor beziehungsweise Akzeptor markierten Biomolekülen beruhend auf dem Prinzip des Fluoreszenz-Resonanz-Energie-Transfers und der Messung der resultierenden Fluoreszenz, dadurch gekennzeichnet, dass metallische Chelat-Komplexe mit Metallionen aus der VII. und VIII. Gruppe der Übergangselemente als Energie-Donoren

in Kombination mit niedermolekularen Fluorophoren mit einem Molekulargewicht zwischen 300 d und 3000 d als Energie-Akzeptoren eingesetzt werden.

Eine bevorzugte Ausgestaltung der Erfindung betrifft einen homogenen Test mit verbesserter Sensitivität zur Bestimmung der Wechselwirkung zwischen mit Donor beziehungsweise Akzeptor markierten Biomolekülen beruhend auf dem Prinzip des Fluoresz-Resonanz-Enzenergie-Transfers und der Messung der resultierenden Fluoreszenz, dadurch gekennzeichnet, dass metallische Chelat-Komplexe mit Metallionen aus der VII. und VIII. Gruppe der Übergangselemente als Energie-Donoren in Kombination mit niedermolekularen Fluorophoren mit einem Molekulargewicht zwischen 300 d und 3000 d als Energie-Akzeptoren eingesetzt werden.

Für den Fachmann ist es nunmehr sehr leicht, basierend auf der vorliegenden Erfindung die für seine Zwecke optimale Kombination von Donor und Akzeptor auszuwählen.

Besonders bevorzugt werden die erfindungsgemäßen, neuartigen, sensitiven FRET-Paare eingesetzt, um molekulare Wechselwirkungen zu bestimmen. Beispiele für solche Wechselwirkungen sind insbesondere hybridisierende Reaktionen von Nukleinsäuren, die Anbindung von Biomolekülen an entsprechende Rezeptoren sowie die Wechselwirkungen zwischen Antigen oder Hapten und Antikörper, oder anderen bioaffinen Bindepaaren z. B. zwischen Lektin und Zucker.

FRET-Paare können jedoch auch eingesetzt werden, um z. B. Abstandsmessungen zwischen Donor- und Akzeptormolekül zu ermöglichen. Veränderungen solcher Molekülabstände können z. B. verwendet werden, um eine enzymatische Aktivität zu dokumentieren. Die Verwendung von metallischen Chelat-Komplexen als Energie-Donoren in Kombination mit niedermolekülen Fluorophoren als Energie-Akzeptoren zur Ermittlung von Wechselwirkungen zwischen Biomolekülen stellt deshalb eine ebenfalls besonders bevorzugte Ausgestaltung dieser Erfindung dar.

Ein besonderer Vorteil der Ruthenium-Komplexe ist die Lebensdauer im Bereich von 50 ns - ca. 10 μs, die eine hohe Repetitionsrate sowie eine kurze Totzeit bei der Messung erlauben. Unter Lebensdauer versteht man die Zeit, die verstreicht, bis die Hälfte der Energie eines FRET-Systems wieder abgestrahlt wurde. Die kurze Lebensdauer der erfindungsgemäßen Farbstoffpaare unter Verwendung von Ruthenium-Komplexen als Donoren ist besonders vorteilhaft, weil repetitiv - also mehrfach - gemessen werden kann. Werden z. B. Europium-Chelat-Komplexe als Donoren eingesetzt, so ist es üblich, ein Meßfenster in einem Zeitbereich von ca. 300 μs und 1 ms zu wählen, wobei meist über einen Zeitraum von ca. 200 μs gemessen wird. Diese Vorgehensweise ist bedingt durch die lange Lebensdauer der angeregten Europium-Komplexe, sodass kürzere Meßfenster nachteilig wären. Für die erfindungsgemäßen Farbstoffpaare, z. B. unter Verwendung von Ruthenium-Komplexen als Donoren ergeben sich dagegen wesentliche Vorteile. Die Ruthenium-Komplexe haben meist eine Lebendauer von ca. 50 ns - ca. 10 μs. Da die niedermolekularen Fluorophore sehr kurze Lebensdauern aufweisen, ist die Lebensdauer des Metall-Chelat-Komplexes entscheidend für das optimale Zeitfenster zur Erfassung der erfindungsgemäßen FRET-Paare. Ein einzelner Meßzyklus kann in ca. 100μs oder kürzer abgeschlossen sein und die Meßzyklen können mehrfach wiederholt werden. Dies führt zu einer deutlichen Verbesserung der Sensitivität. FRET-Farbstoffpaare mit Ruthenium-Komplexen als Donoren, die Lebenszeiten von 50 ns bis 10 μs haben, sind deshalb besonders zu bevorzugen. Als ganz besonders bevorzugt haben sich Paare mit einer Lebenszeit von 100 ns bis 8 μs erwiesen.

Es ist bevorzugt, dass die erfindungsgemäßen FRET-Paare eingesetzt werden, um z. B. die Anwesenheit oder die Konzentration eines Biomoleküles zu ermitteln. Besonders bevorzugt wird dabei ein Partner des FRET-Paares an einen Bindepartner für besagtes Biomolekül gebunden während ein anderer Partner des FRET-Paares direkt oder indirekt an das besagte Biomolekül gebunden ist oder wird. Ein einfaches derartiges System verwendet, z. B., mit Metall-Chelat-markiertes Antigen und Fluorophor-markierte Antikörper (oder umgekehrt). Entsprechende Modelle und Beispiele sind im Methodenteil angeführt.

Wie eingangs erwähnt, ist es ein besonderer Vorzug von FRET-Systemen, speziell von TR-FRET-Systemen, dass die Bestimmung von Wechselwirkungen zwischen Biomolekülen ohne Waschschritte, d.h., in sogenannten homogenen Bestimmungsverfahren erfolgen kann. Homogene Bestimmungsverfahren unter Verwendung der erfindungsgemäßen Farbstoffkombinatione sind deshalb besonders bevorzugt.

Die beiden Partner der erfindungsgemäßen Farbstoffpaare, Metall (Übergangselemente der Gruppe VII oder VIII)-Chelat-Donor einerseits, sowie niedermolekulares Fluorophor andererseits lassen sich in bekannter Weise, wie z. B. in EP 178 450 und in EP 772 616 ("Hydrophile Metallkomplexe") bzw. in EP 567 622 oder EP 747 447 beschrieben an Biomoleküle koppeln. Diese Kopplungsprodukte sind gut wasserlöslich und sehr stabil unter Transport- oder Lagerbedingungen. Sie eigenen sich deshalb auch in hervorragender Weise um Test- oder Reagenz-Kits zusammenzustellen, welche den Nachweis eines Analyten in einer Probe ermöglichen, wobei mindestens ein mit einem Metall-Chelat-Komplex markiertes Biomolekül, sowie mindestens ein weiteres Biomolekül welches mit einem niedermolekularen Fluorophor markiert ist, in diesem Reagenz-Kit enthalten sind.

Eine bevorzugte Ausgestaltung der Erfindung ist ein Reagenz oder eine Reagenzkombination zur Bestimmung der Wechselwirkung zwischen den Partnern eines bioaffinen Bindepaares, dadurch gekennzeichnet, dass ein Partner eines bioaffinen Bindepaares mit einem Metall-Chelat-Komplex mit Metallionen aus der VII. und VIII. Gruppe der Übergangselemente und ein anderer Partner dieses bioaffinen Bindepaares mit einem niedermolekularen Fluorophor mit einem MW zwischen 300 < 3000 d markiert ist.

Eine weitere bevorzugte Ausführungsform der Erfindung ist ein Reagenz-Kit, der neben den mit den FRET-Partnern markierten Biomolekülen auch noch weitere nützliche Reagenzien enthalten wird, welche für die Durchführung der Analytbestimmung dienlich sind, beispielsweise können dies bestimmte Puffer oder Kontrollreagenzien sein.

Die folgenden Beispiele, die zitierten Publikationen, das Sequenzprotokoll, die Formeln und die Abbildung erläutern die Erfindung, deren Schutzumfang sich aus den Patentansprüchen ergibt, weiter. Die beschriebenen Verfahren sind als Beispiele zu verstehen, die auch noch nach Modifikationen den Gegenstand der Erfindung beschreiben.

Figuren

Abbildung 1: Schema der Messvorrichtung

Schematische Darstellung der im Rahmen der vorliegenden Erfindung verwendeten Messapparatur für die zeitaufgelöste Fluoreszenzmessung. Diese Messanordnung und ihre Verwendung wird in den folgenden Beispielen genauer ausgeführt und erläutert.

Beispiele:

*Verwendete Abkürzungen*

| | |
|---|---|
| DADOO = | 1,8-Diamino-3,6-dioxaoctan |
| batho = | Bathophenanthrolin-disulfonsäure |
| bpy = | 2,2'-Bipyridyl-4-methyl-4'-butylcarbonsäure |
| APC = | Allophycocyanin |
| HA = | Humanes Hämagglutinin |
| HA-Peptid = | YPYDVPDYA |
| OSu = | O-Succinimid |
| Strept. = | Streptavidin |
| Ru = | Ruthenium |
| Eu = | Europium |

**Beispiel 1: Synthesen und Markierungen von Biomolekülen**

***a) Synthese von Ru(batho)$_2$bpy-OSu***

50 mg (3*10$^{-5}$ mol) Ru(batho)$_2$bpy werden in DMF gelöst, mit 12 mg (6*10$^{-5}$ mol) EDC (N-(3-Dimethylaminopropyl)-N'-ethylcarbodiimid-hydrochlorid) sowie 7 mg (6*10$^{-5}$ mol) NHS (N-Hydroxysuccinimid) versetzt und bei Raumtemperatur über Nacht gerührt. Durch Zugabe von Aceton fällt das Reaktionsprodukt als Niederschlag aus. Dieser wird abfiltriert und im Vakuum getrocknet. Die Aufreinigung erfolgt mittels HPLC. Das Endprodukt wurde mittels MALDI-MS analysiert und entspricht Formel III.

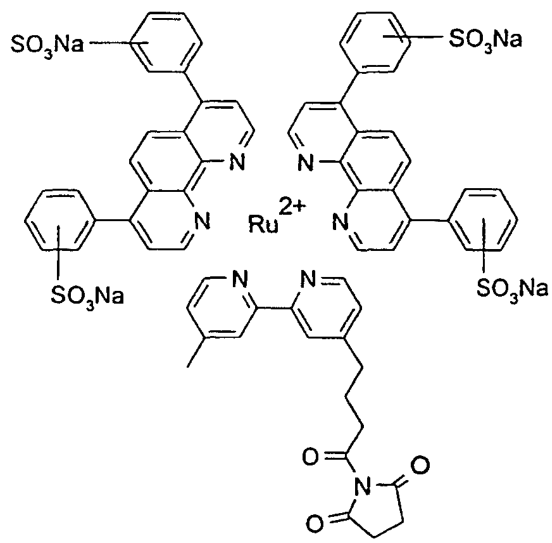

Formel III: Ru(batho)$_2$bpy-OSu

***b) Synthese von Streptavidin-Ru(batho)$_2$bpy***

10 mg (1.9*10$^{-7}$ mol) Streptavidin werden in 1 ml 0.1 N $NaHCO_3$-Lösung gelöst und mit 2 mg (1.3*10$^{-6}$ mol) Ru(batho)$_2$bpyOSu (gelöst in 0.5 ml einer wässrigen 0.1 N $NaHCO_3$-Lösung) tropfenweise versetzt. Man rührt 1 Stunde bei Raumtemperatur und chrommatographiert den Ansatz über eine Sephadex LH20-Säule (Eluent 0.1 N $NaHCO_3$-Lösung). Die Produktfraktionen, d. h. die Fraktionen, die das markierte Streptavidin (Protein o.D. 280 nm) enthalten, werden über Nacht gegen $H_2O$ dialysiert und anschließend gefriergetrocknet.

Der Markierungsgrad wurde über Vergleich der Absorption bei 280 nm (Protein) und 460 nm ($Ru^{2+}$-Komplex) bestimmt und mit ca. 3,6 $Ru^{2+}$-Komplexe pro Streptavidin ermittelt. Streptavidin-Ruthenium-Konjugate mit durchschnittlich 3 bis 5 $Ru^{2+}$-Komplexen pro Streptavidinmolekül sind für die Sensitivitätsbestimmung geeignet.

***c) Kopplung von Ru(batho)2bpy an MAK-anti-HA***

300 µl (3 mg) MAK-Anti-HA werden mit 1 mg (0.65*10$^{-6}$ mol) Ru(batho)$_2$bpyOSu (gelöst in 1.0 ml einer wässrigen 0.1 N $NaHCO_3$-Lösung) tropfenweise versetzt. Man rührt 1 Stunde bei Raumtemperatur und chrommatographiert den Ansatz über eine LH20-Säule (Eluent 0.1 N $NaHCO_3$-Lösung). Die Produktfraktionen, d. h. die Fraktionen, die den markierten Antikörper enthalten (o. D. 280 nm) werden vereinigt und als 0.1 N $NaHCO_3$-Lösungen eingefroren.

Im vorliegenden Experiment waren durchschnittlich etwa 7 Ruthenium-Komplexe pro Antikörper gebunden.

***d) Herstellung von Biotin-JA286***

10.2 mg (3*10$^{-5}$ mol) Biotin-DADOO sowie 17.2 mg (3*10$^{-5}$ mol) JA 286 werden in 1 ml DMF gelöst, mit 15 µl Triethylamin versetzt und auf 0°C abgekühlt. Nach der Zugabe von 5 µl DECP (Diethyl-cyanphosphonat) wird der Ansatz 1 Stunde bei 0°C und anschließend bei Raumtemperatur über Nacht gerührt. Nach dem Einengen bis zur Trockne wird das Rohprodukt mittels HPLC aufgereinigt. Das Endprodukt wurde mittels MALDI-MS charakterisiert und entspricht Formel IV:

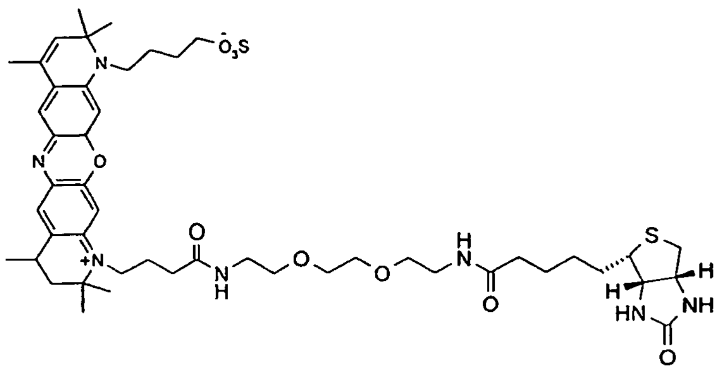

Formel IV: Biotin-JA286

*e) Synthese von Biotin-JA198*

5 mg (5.3*$10^{-6}$ mol) JA198-OSu sowie 2 mg (5.3*$10^{-6}$ mol) Biotin-DADOO werden in 800 µl Phosphatpuffer pH 7.5 gelöst und unter Lichtausschuß bei Raumtemperatur über Nacht gerührt. Anschließend wird der Ansatz über die HPLC aufgereinigt. Das Produkt wurde mit ESI-MS geprüft und entspricht Formel V:

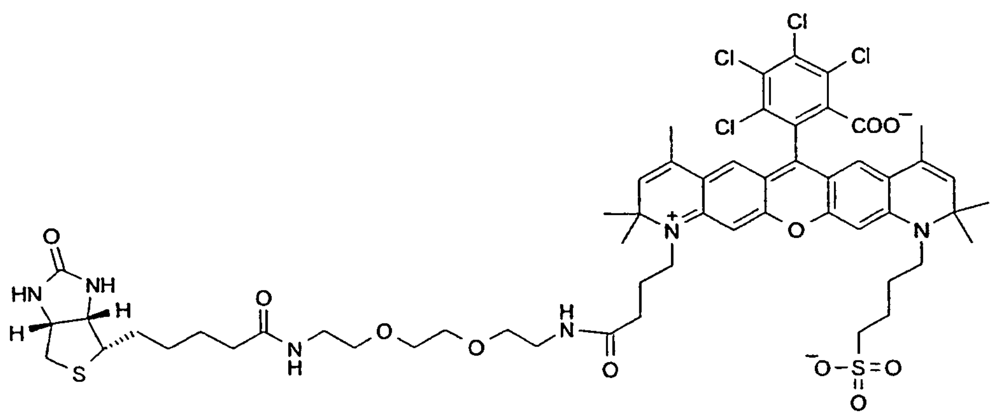

Formel V: Biotin-JA198

*f) Kopplung von JA133 an das synthetische HA-Peptid*

5 mg (4.5*$10^{-6}$ mol) HA-Peptid (YPYDVPDYA=SEQ ID NO: 1)werden in 1 ml Acetonitril/Phosphatpuffer pH7.5 (1:1) gelöst und unter Rühren mit einer Lösung aus 4 mg (4.5*$10^{-6}$ mol) JA133-OSu und 500 µl Acetonitril versetzt. Der Ansatz wird bei Raumtemperatur über Nacht gerührt.

Nach dem Einengen im Vakuum bis zur Trockne erfolgt die Aufreinigung mittels HPLC. Das Produkt wurde mittels ESI-MS geprüft und entspricht Formel VI:

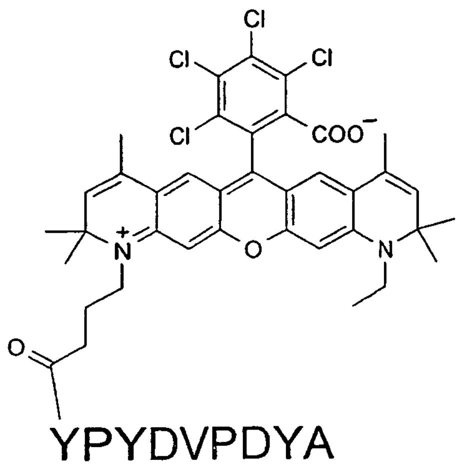

Formel VI: HA-Peptid-JA133

**Beispiel 2: Meßvorrichtung und deren Beschreibung**

Die im Rahmen der vorliegenden Erfindung verwendete Messapparatur für die zeitaufgelöste Fluoreszenzmessung wird im folgenden beschrieben und anhand eines Schemas (Abbildung 1) erläutert.

Die gepulste Lichtquelle (1) - Stickstofflaser oder Farbstofflaser - regt mit Lichtpulsen geeigneter Wellenlänge (13) den Donor-Marker der Messprobe (3) an, wobei die Lichtpulsbreite t = 0.7 ns viel kürzer als die Abklingzeit des einen der fluoreszierenden Markers ist. Durch Energie-Übertragung wird dann der Akzeptor-Marker zur Fluoreszenz angeregt. Diese Fluoreszenzstrahlung (14) wird mit einer Optik (4,6) durch einen optischen Filter (Kantenfilter / Bandpassfilter) (5), der die Emissionswellenlänge des Akzeptor-Markers durchläßt, auf die Photokathode eines Photomultipliers (7) geführt. Die einzelnen detektierten Photonen erzeugen Strompulse, die nach Verstärkung (8) und Normierung (9) digital gezählt werden (10) (Photon-Counting Methode). Über eine Quarzplatte (2) wird ein Bruchteil der anregenden Lichtes (15) zu einer Photodiode (11) abgelenkt, die eine Torschaltung (12) steuert, welche nach einer einstellbaren Verzögerungszeit - vorzugsweise von 1 µs - den Zähler (10) startet und nach einer einstellbaren Öffnungszeit des Messfensters - vorzugsweise von 100 µs - den Zählprozess wieder stoppt. Die Verzögerungszeit wird so gewählt, dass in ihr Streulichteffekte sowie die Backgroundfluoreszenz praktisch vollständig abgeklungen sind. Auf diese Art wird die Anzahl gezählter Strompulse proportional zu der Markerfluoreszenzintensität, welche separat vom Background gemessen wird. Die Durchführung entsprechender Messungen ist in den Beispielen 3 und 4 für zwei neue TR-FRET-Paare im Detail beschrieben. Die anderen in Beispiel 5 angeführten weiteren neuen FRET-Paare wurden analog vermessen, bzw., die Europium-APC-Systeme des Beispiels 5, mit aus dem Stand der Technik bekannten Geräten und Methoden quantifiziert.

**Beispiel 3: Durchführung einer Empfindlichkeitsmessung eines FRET-Systems mit "$Ru^{2+}$-Komplex" als Donor und mit JA-286 als fluorophorem Akzeptor.**

In die Messküvette werden 400 µl einer Lösung (10 mM Na-Phosphat, 150 mM NaCl, pH 7.2) zu 100 nM Streptavidin - markiert mit dem "$Ru^{2+}$-Komplex" - und zu 300 nM Biotin-DADOO-JA-286 pipettiert.

Die Messung erfolgt mit der in Beispiel 2 beschriebenen und in Abbildung 1 schematisch dargestellten Apparatur. Der Donor wird durch einen 460nm Farbstofflaser angeregt. Die Lichtpulsdauer ist = 1 ns. Die Fluoreszenz des Systems wird durch die Kombination optischer Kantenfilter KV550 + RG645 mit dem Photomultplier gemäß der beschriebenen Versuchsanordnung in der Photon-Counting-Technik detektiert.

Die Verzögerungszeit wird auf 1 µs eingestellt und das Messfenster beträgt 100 µs. Der Background wird durch eine separate Messung des Puffers unter den gleichen Bedingungen ermittelt. Die Empfindlichkeit (untere Nachweisgrenze) wird durch eine Grenzkonzentration angegeben, die durch die folgende Beziehung bestimmt ist:

$$c_0 = \frac{2B}{S} c_S,$$

wobei $c_0$ die Grenzkonzentration in [M], B den Background in [Counts], S das Signal in [Counts] und $c_S$ die Probenkonzentration in [M] bedeuten.

Die untere Nachweisgrenze für dieses System liegt bei $7.4 \cdot 10^{-14}$ M Streptavidin.

**Beispiel 4: Durchführung der Empfindlichkeitsmessung eines FRET-Systems mit "$Ru^{2+}$-Komplex" als Donor und mit JA-133 als Akzeptor bei einer Antigen-Antikörper Reaktion.**

Die FRET-Partner in diesem Beispiel sind der $Ru^{2+}$-Komplex aus Beispiel 1a) als Donor und JA-133 als Akzeptor. Ein monoklonaler Antikörper gegen HA wird mit dem Donor, wie unter 1c) beschrieben, markiert. Das "Antigen" (HA-Peptid) wird mit dem Akzeptor gemäß 1f) markiert. Durch die Antigen-Antikörper Reaktion wird das Donor-Akzeptor-Paar in ausreichender Nähe zueinander gebracht, sodass FRET möglich und messbar wird.

Nach 10 Minuten Inkubation bei Zimmertemperatur werden 400 µl einer Lösung (10 mM Na-Phosphat, 150 mM NaCl, pH 7.2) zu 100 nM anti-HA - markiert mit dem "$Ru^{2+}$-Komplex" - und zu 100 nM HA-JA-133 in die Messküvette pipettiert.

Die Messung erfolgt mit der unter Beispiel 2 beschriebenen Apparatur. Der Donor wird bei 460 nm durch einen 460nm Farbstofflaser angeregt. Die Lichtpulsdauer ist = 1 ns. Die Fluoreszenz des Systems wird durch die Kombination optischer Kantenfilter KV550 + RG630 mit dem Photomultplier gemäß der Versuchsanordnungsbeschreibung in der Photon-Counting-Technik detektiert.

Die Verzögerungszeit wird auf 1 µs eingestellt und das Messfenster beträgt 100 µs. Der Background wird durch eine separate Messung des Puffers unter den gleichen Bedingungen ermittelt. Die Empfindlichkeit wird durch eine Grenzkonzentration $c_0$ in [Mol/Liter] wie in Beispiel 1 angegeben.
Die Empfindlichkeit bei diesem System beträgt $7.2 \cdot 10^{-14}$ M an HA.

**Beispiel 5: Zusammenstellung einiger neuer FRET-Paare und Vergleich mit FRET-Paaren aus dem Stand der Technik**

***a)* TR-FRET-Paar 1: Streptavidin-Ru(batho)2bpy/ Biotin-APC**

Das markierte Streptavidin aus Beispiel 1b) wurde in einer Konzentration von 100nM das APC-Biotin (kommerzielles Produkt: APC-XL-Biotin von Europa Bioproducts Ltd., Cambridge, GB) mit 300 nM eingesetzt. Die Anregungswellenlänge ($\lambda_{EX}$) lag bei 460 nm die Emissions- oder Meßwellenlänge ($\lambda_{EM}$) bei 634 nm. Mit diesem Farbstoff- und Reagenzpaar wurde die untere Nachweisgrenze mit 4.1 x $10^{-13}$ Mol/Liter($\triangleq$ M) ermittelt.

***b*) R-FRET-Paar 2: Streptavidin-Ru(batho)2bpy/ Biotin-JA198**

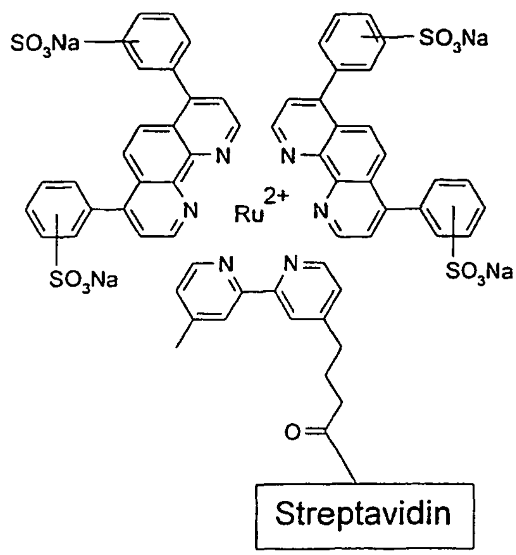

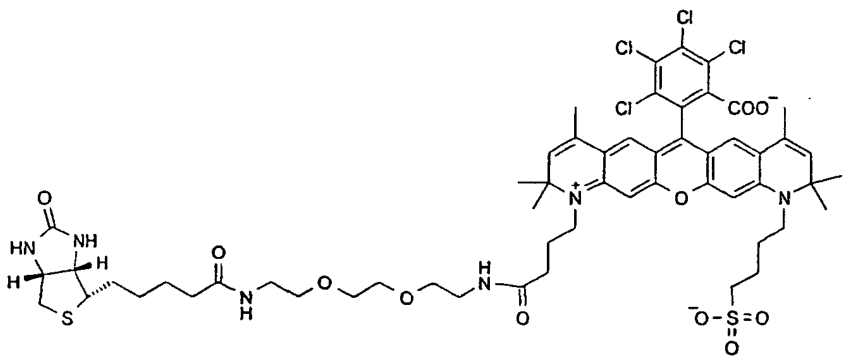

***c)*** **TR-FRET-Paar 3:Streptavidin-Ru(batho)2bpy/ Biotin-JA286**

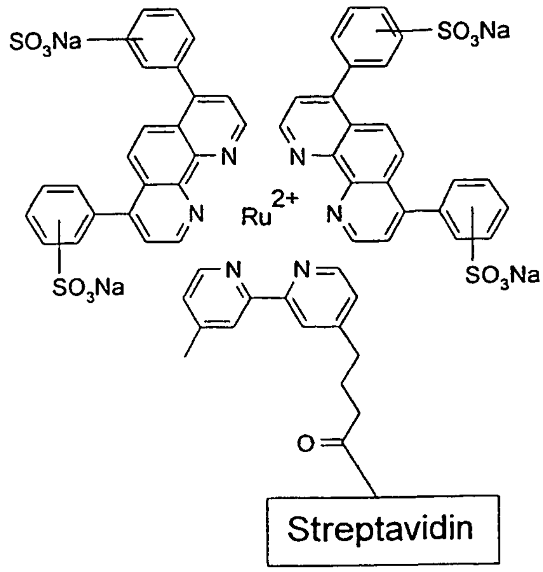

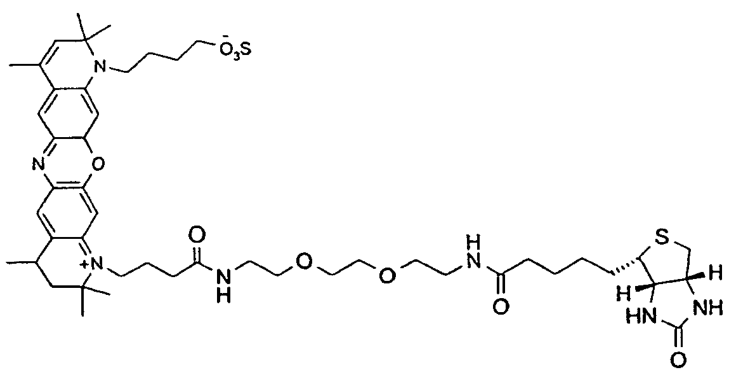

***d)*** **TR-FRET-Paar 4: MAK-anti-HA-Ru(batho)2bpy/HA-JA133**

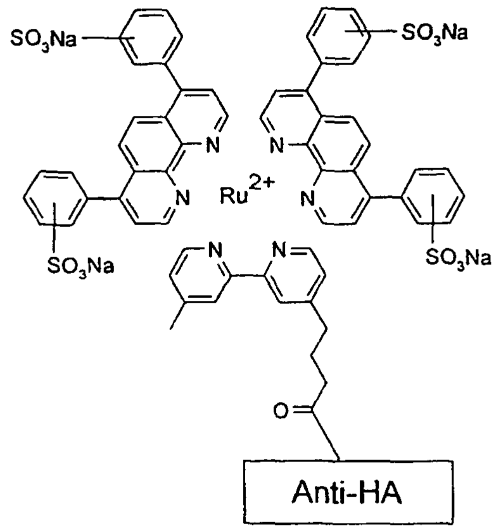

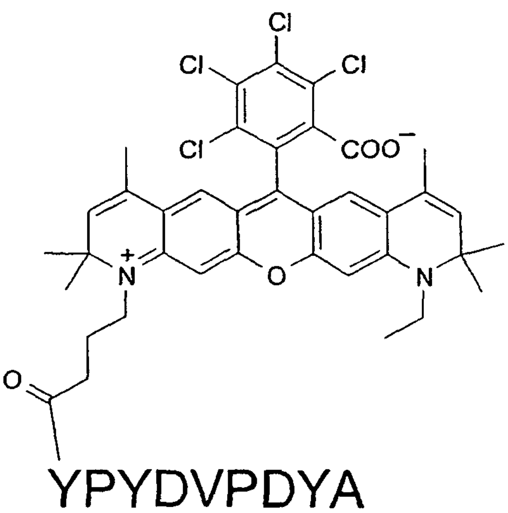

*e) + f)* Weitere FRET-Paare unter Verwendung kommerziell verfügbarer Einsatzstoffe.

Für die FRET-Paare 5 und 6 der Tabelle 1 wurden als Donoren kommerziell erhältliche, mit Europium-markierte Streptavidinderivate von Wallac, Oy, eingesetzt (Strept-Eu0062: AD0062 Streptavidin-W1024; Strept-Eu0060: AD0060 Streptavidin-W8044). Als Akzeptor wurde das in Beispiel 5a) angeführte und kommerziell erhältliche Biotin-APC eingesetzt.

Tabelle 1: Untere Nachweisgrenze für einige untersuchte FRET-Paare

| Nr. | System | Konzentration [µM] Strept. / Biotin | $\lambda_{EX}$ [nm] | $\lambda_{EM}$ [nm] | Untere Nachweisgrenze (UNG)[Mol/Liter] |
|---|---|---|---|---|---|
| 1 | Strept.-Ru(batho)$_2$bpy / Biotin-APC | 0.100 / 0.300 | 460 | 634 | $4.1\cdot10^{-13}$ |
| 2 | Strept.-Ru(batho)$_2$bpy / Biotin- JA198 | 0.150 / 0.150 | 460 | 660 | $2.6\cdot10^{-13}$ |
| 3 | Strept.-Ru(batho)$_2$bpy / Biotin- JA286 | 0.100 / 0.300 | 460 | 703 | $7.4\cdot10^{-14}$ |
| 4 | Anti-HA-Ru-(batho)$_2$bpy / HA-JA133 | 0.100 / 0.100 | 460 | 631 | $7.2\cdot10^{-14}$ |
| 5(#) | Strept.-Eu0062 / Biotin-APC | 0.030 / 0.090 | 337 | 660 | $2.5\cdot10^{-11}$ |
| 6(#) | Strept.-Eu0060 / Biotin-APC | 0.030 / 0.090 | 337 | 660 | $7.3\cdot10^{-12}$ |

Sensitivität gemessen mit:

1 µs Delay und 100 µs Meßfenster, bestimmt gemäß: $c_0 = \frac{2B}{S} c_S$ [M],

wobei $c_0$: untere Grenzkonzentration in [M], $c_s$: verwendete Konzentration in [M], B: Hintergrund (vom Puffer) in [counts], S: Signal in [counts].

(#): Meßfenster: 400 µs – wie im Stand der Technik für Eu-APC-Komplexe beschrieben.

Aus Tabelle 1 geht klar hervor, dass die neuartigen FRET-Paare den im Stand der Technik bekannten Systemen zumindest gleichwertig oder überlegen sind. Das erfindungsgemäße FRET-Paar Nr. 3 hat eine UNG von $7.4\cdot10^{-14}$ welche deutlich tiefer liegt, als die UNG für die Beispiele Nr. 5 oder Nr. 6, gemäß Stand der Technik.

**Referenzliste**

EP 178 450
EP 567 622
EP 747 447
EP 076 695

EP 772 616
EP 439 036
EP 1 054 039
US 5,998,146
WO 00/47693

Blomberg, et al., Clinical Chemistry 45 (1999) 855ff.
French, et al., SPIE BiOS in Proc. SPIE v 3259 (1998) 209-218
French, et al., SPIE BiOS in Proc. SPIE v 3603 (1999) 272-280
Hemmilä, Chemical Analysis 117
John Wiley&Sons, Inc., (1991)135-139
Joun et al., Analytical Biochemistry 232 (1995) 24-30
Mujumdar, et al., Bioconjugate Chem. 7, 1996, 356-362
Van der Meer, et al., Resonance Energy Transfer VCH (1994)

**Patentansprüche**

1. Methode zur Bestimmung der Wechselwirkung zwischen mit Donor beziehungsweise Akzeptor markierten Biomolekülen beruhend auf dem Prinzip des Fluoreszenz-Resonanz-Energie-Transfers und der Messung der resultierenden Fluoreszenz, **dadurch gekennzeichnet, dass** metallische Chelat-Komplexe mit Metallionen aus der VII. und VIII. Gruppe der- Übergangselemente als Energie-Donoren in Kombination mit niedermolekularen Fluorophoren mit einem Molekulargewicht zwischen 300 d und 3000 d als Energie-Akzeptoren eingesetzt werden.

2. Methode gemäß Anspruch 1, **dadurch gekennzeichnet, dass** die niedermolekularen Fluorophore ein Absorptionsmaximum zwischen 600 nm und 750 nm aufweisen.

3. Methode gemäß Anspruch 1 oder 2, **dadurch gekennzeichnet, dass** die niedermolekularen Fluorophore ein Molekulargewicht < 2000 d aufweisen.

4. Methode gemäß einem der Ansprüche 1 bis 3, **dadurch gekennzeichnet, dass** die Fluorophore ausgewählt werden aus der die Xanthene, Cyanine, Rhodamine und Oxazine umfassenden Gruppe.

5. Methode gemäß einem der Ansprüche 1 bis 4, **dadurch gekennzeichnet, dass** als Fluorophor ein Rhodamin der folgenden allgemeinen Formel I

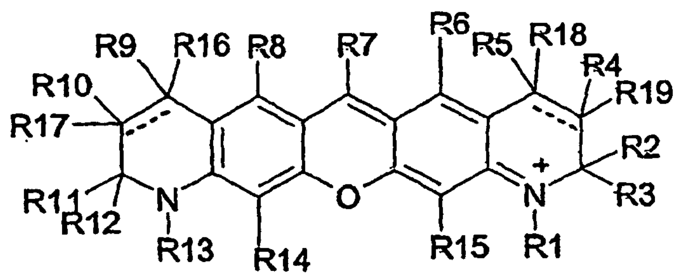

X-

(Formel I)

verwendet wird, worin R1 und R13 gleich oder verschieden sind und bedeuten: Wasserstoff, Alkyl mit 1 bis 20 Kohlenstoffatomen, Polyoxyhydrocarbyl-Einheiten, Phenyl, Phenylalkyl mit 1 bis 3 Kohlenstoffatomen in der Alkylkette, worin die Alkyloder/und Phenylreste durch eine oder mehrere Hydroxy-, Halogen-, Sulfo-, Carboxy- oder Alkoxycarbonylgruppen, worin Alkozy 1 bis 4 Kohlenstoffatome besitzen kann, substituiert sein können;
R7 eine durch mindestens ein Halogen substituierte Alkylgruppe mit 1 bis 20, vorzugsweise 1 bis 7 Kohlenstoffatomen oder eine Phenylgruppe, die durch eine in o-Stellung _zu dem an das pentacyclische Ringsystem gebundene Kohlenstoffatom befindliche Carboxy- oder Alkoxycarbonylgruppe und mit mindestens 1 Halogen substituiert ist, und worin Alkoxy 1 bis 4 Kohlenstoffatome besitzen kann, oder eine Carboxyalkylgruppe oder eine Carboxymethylenoxy-alkyloxygruppe bedeutet;
die beiden strichliniert eingezeichneten Bindungen bedeuten, dass die beiden durch die strichlinierte Bindung verbundenen Kohlenstoffatome durch eine Einfach- oder Doppelbindung miteinander verbunden sein können; wobei R14, R15 und die übrigen, nicht mit spezifischen Symbolen bezeichneten Positionen des pentacyclischen Grundkörpers durch Alkylgruppen mit 1 bis 20 Kohlenstoffatomen substituiert sein können; wobei X- ein Gegenion ist und wobei zumindest einer der Reste R1, R7 oder/und R 13 an ein Biomoleküfgekoppelt ist.

6. Methode gemäß Anspruch 5, **dadurch gekennzeichnet, dass** der Substituent R7 ein elektronenziehender Rest ist

7. Methode gemäß einem der Ansprüche 1 bis 4, **dadurch gekennzeichnet, dass** als Fluorophor ein Oxazin der folgenden allgemeinen Formel II

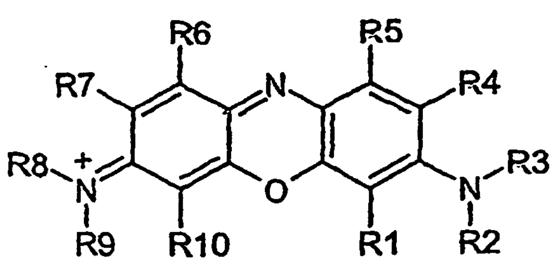

(Formel II)

verwendet wird, worin R1, R4, R5, R6, R7, R10 Wasserstoff Alkyl, Hydroxy, Halogen, Carboxyl, Sulfonyl oder Amino bedeutet und
R2, R3, Wasserstoff, Alkyl, Alkony, Polyoxyhydroxycarbonyleinheiten, Phenyl, Phenylalkyl bedeutet, die durch Hydroxy, Sulfonyl, Carboxy, Amino, Alkoxycarbonyl substituiert sein können, wobei R2 mit R1 oder R3 mit R4 eine gesättigte oder ungesättigte C4- oder C5-Brücke bilden kann und
R8, R9, Wasserstoff, Alkyl, Alkoxy, Polyoxyhydroxycarbonyleinheiten, Phenyl, Phenylalkyl bedeutet, die durch Hydroxy, Sulfonyl, Carboxy, Amino, Alkoxycarbonyl substituiert sein können, wobei R2 mit R1 oder R3 mit R4 eine gesättigte oder ungesättigte C4- oder C5-Brücke bilden kann und
wobei mindestens eine der Reste R2, R3, R8 oder R9 einen nicht-brückebildenden Rest darstellt, der an ein Biomolekül gekuppelt ist und wobei mindestens einer der Reste R2, R3, R8 und R9 einen brückebildenden Rest darstellt, der gegebenenfalls durch Alkyl substituiert sein kann darstellt.

**8.** Methode gemäß einem der vorstehenden Ansprüche, **dadurch gekennzeichnet, dass** der Metall-Chelat-Komplex ein Ruthenium-Komplex ist.

**9.** Methode gemäß einem der Ansprüche 1 bis 8 **dadurch gekennzeichnet, dass** die Messung der Fluoreszenz zeitaufgelöst erfolgt.

**10.** Methode gemäß einem der Ansprüche. 1 bis 8 **dadurch gekennzeichnet, dass** die Messung der Fluoreszenz mittels der Phasenmodulationstechnik erfolgt.

**11.** Verwendung von Metall-Chelat-Komplexen mit Metallionen aus der VII. und VIII. Gruppe der Übergangselemente als Fluoreszenz-Resonanz-Energie-Donoren in Kombination mit niedermolekularen Fluorophoren mit einem Molekulargewicht zwischen 300 d und 3000 d in einem Verfahren zum Nachweis einer Wechselwirkung mindestens zweier Biomoleküle in einer Probe.

**12.** Verwendung nach Anspruch 11, **dadurch gekennzeichnet, dass** das Verfahren nach dem Prinzip eines homogenen Bindetests durchgeführt wird.

**13.** Reagenz-Kit zum Nachweis eines Analyten in einer Probe, **dadurch gekennzeichnet, dass** mindestens ein mit einem Metall-Chelat-Komplex mit Metallionen aus der VII. und VIII. Gruppe der Übergangselemente markiertes Biomolekül, sowie mindestens ein weiteres Biomolekül welches mit einem niedermolekularen Fluorophor mit einem Molekulargewicht zwischen 300 d und 3000 d markiert ist, enthalten ist.

**14.** Reagenzkombination zur Bestimmung der Wechselwirkung zwischen den Partnern eines bioaffinen Bindepaares, **dadurch gekennzeichnet, dass** ein Partner des bioaffinen Bindepaares mit einem Metall-Chelat-Komplex mit Metallionen aus der VII. und VIII. Gruppe der Übergangselemente und ein anderer Partner des bioaffinen Bindepaares mit einem niedermolekularen Fluorophor mit einem Molekulargewicht zwischen 300 d und 3000 d markiert ist.

**15.** Verfahren zur Bestimmung der Wechselwirkung zwischen mit Donor beziehungsweise Akzeptor markierten Biomolekülen beruhend auf dem Prinzip des Fluoreszenz-Resonanz-Energie-Transfers und der Messung der resultierenden Fluoreszenz, **dadurch gekennzeichnet, dass** metallische Chelat-Komplexe mit Metallionen aus der VII. und VIII. Gruppe der Übergangselemente als Energie-Donoren in Kombination mit niedermolekularen Fluorophoren mit einem Molekulargewicht zwischen 300 d und 3000 d als Energie-Akzeptoren eingesetzt werden.

## Claims

**1.** Method for determining the interaction between biomolecules labelled with a donor or acceptor based on the principle of fluorescence resonance energy transfer and measurement of the resulting fluorescence **characterized in that** metallic chelate complexes containing metal ions from the VIIth and VIIIth group of the transition elements are used as energy donors in combination with low-molecular fluorophores having a molecular weight between 300Da and 3000 Da as energy acceptors.

**2.** Method as claimed in claim 1, **characterized in that** the low-molecular fluorophores have an absorption maximum between 600 nm and 750 nm.

3. Method as claimed in claim 1 or 2, **characterized in that** the low-molecular fluorophores have a molecular weight of < 2000 Da.

4. Method as claimed in one of the claims 1 to 3, **characterized in that** the fluorophores are selected from the group comprising xanthenes, cyanins, rhodamines and oxazines.

5. Method as claimed in one of the claims 1 to 4, **characterized in that** a rhodamine of the following general formula I is used as the fluorophore

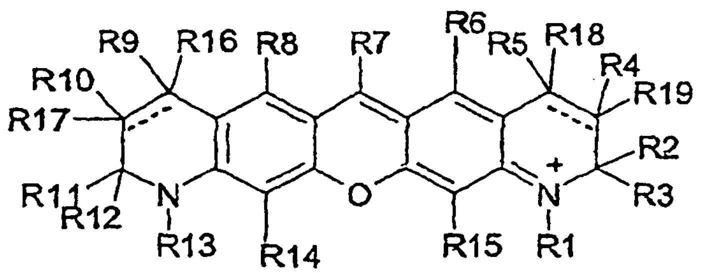

X-

(formula I)

in which R1 and R13 are the same or different and denote: hydrogen, alkyl with 1 to 20 carbon atoms, polyoxyhydrocarbyl units, phenyl, phenylalkyl with 1 to 3 carbon atoms in the alkyl chain wherein the alkyl or/and phenyl residues can be substituted by one or more hydroxy, halogen, sulfo, carboxy or alkoxycarbonyl groups in which alkoxy can have 1 to 4 carbon atoms; R7 denotes an alkyl group substituted by at least one halogen with 1 to 20, preferably 1 to 7 carbon atoms or a phenyl group which is substituted by a carboxy or alkoxycarbonyl group in the o-position relative to the carbon atom bound to the pentacyclic ring system and by at least one halogen, and wherein alkoxy can have 1 to 4 carbon atoms, or a carboxyalkyl group or a carboxymethyleneoxy-alkyloxy group; the two bonds marked by the dashed lines mean that the two carbon atoms linked by the dashed bond can be linked together by a single or double bond; wherein R14, R15 and the other positions of the pentacyclic basic structure that are not labelled with specific symbols can be substituted by alkyl groups with 1 to 20 carbon atoms; wherein X is a counterion and wherein at least one of the residues R1, R7 or/and R13 is coupled to a biomolecule.

6. Method as claimed in claim 5, **characterized in that** the substituent R7 is an electron-attracting residue.

7. Method as claimed in one of the claims 1 to 4, **characterized in that** an oxazine of the following general formula II is used as the fluorophore

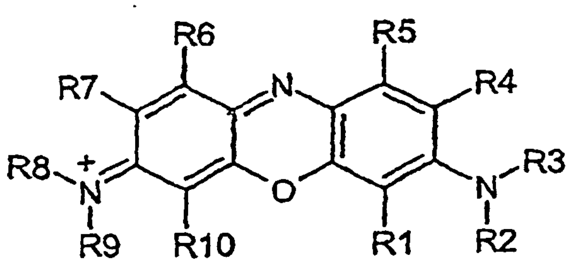

(formula II)

in which R1, R4, R5, R6, R7, R10 denote hydrogen, alkyl, hydroxy, halogen, carboxyl, sulfonyl or amino and R2, R3 denote hydrogen, alkyl, alkoxy, polyoxyhydroxycarbonyl units, phenyl, phenylalkyl which can be substituted

by hydroxy, sulfonyl, carboxy, amino, alkoxycarbonyl, in which R2 and R1 or R3 and R4 can form a saturated or unsaturated C4 or C5 bridge and
R8, R9 denote hydrogen, alkyl, alkoxy, polyoxyhydroxycarbonyl units, phenyl, phenylalkyl which can be substituted by hydroxy, sulfonyl, carboxy, amino, alkoxycarbonyl, in which R2 and R1 or R3 and R4 can form a saturated or unsaturated C4 or C5 bridge and
wherein at least one of the residues R2, R3, R8 or R9 represents a non-bridge forming residue that is coupled to a biomolecule and wherein at least one of the residues R2, R3, R8 and R9 represents a bridge-forming residue which can be optionally substituted by alkyl.

**8.** Method as claimed in one of the previous claims, **characterized in that** the metal chelate complex is a ruthenium complex.

**9.** Method as claimed in one of the claims 1 to 8, **characterized in that** a time-resolved measurement of fluorescence is carried out.

**10.** Method as claimed in one of the claims 1 to 8, **characterized in that** the fluorescence is measured by means of the phase modulation technique.

**11.** Use of metal chelate complexes containing metal ions from the VIIth and VIIIth group of the transition elements as fluorescence resonance energy donors in combination with low-molecular fluorophores having a molecular weight between 300 Da and 3000 Da in a method for detecting an interaction of at least two biomolecules in a sample.

**12.** Use as claimed in claim 11, **characterized in that** the method is carried out according to the principle of a homogeneous binding test.

**13.** Reagent kit for detecting an analyte in a sample, **characterized in that** it contains at least one biomolecule labelled with a metal chelate complex containing metal ions from the VIIth and VIIIth group of the transition elements and at least one other biomolecule which is labelled with a low-molecular fluorophore having a molecular weight between 300 Da and 3000 Da.

**14.** Reagent combination for determining the interaction between partners of a bioaffine binding pair, **characterized in that** one partner of the bioaffine binding pair is labelled with a metal chelate complex containing metal ions from the VIIth and VIIIth group of the transition elements and another partner of the bioaffine binding pair is labelled with a low-molecular fluorophore having a molecular weight between 300 Da and 3000 Da.

**15.** Method for determining the interaction between biomolecules labelled with a donor or acceptor based on the principle of fluorescence resonance energy transfer and measurement of the resulting fluorescence **characterized in that** metallic chelate complexes containing metal ions from the VIIth and VIIIth group of the transition elements are used as energy donors in combination with low-molecular fluorophores having a molecular weight between 300 Da and 3000 Da as energy acceptors.

## Revendications

**1.** Procédé pour déterminer l'interaction entre des biomolécules marquées par un donneur, respectivement par un accepteur, basé sur le principe du transfert d'énergie de fluorescence-résonance, et sur la mesure de la fluorescence résultante, **caractérisé en ce que** des complexes chélate métallique ayant des ions métalliques provenant du groupe VII et VIII des éléments de transition, sont mis en oeuvre en tant que donneurs d'énergie, en combinaison avec des fluorophores de faible masse moléculaire, ayant une masse moléculaire comprise entre 300 d (daltons) et 3 000 d en tant qu'accepteurs d'énergie.

**2.** Procédé selon la revendication 1, **caractérisé en ce que** les fluorophores de faible masse moléculaire présentent un maximum d'absorption compris entre 600 nm et 750 nm.

**3.** Procédé selon la revendication 1 ou 2, **caractérisé en ce que** les fluorophores de faible masse moléculaire présentent une masse moléculaire inférieure à 2 000 d.

**4.** Procédé selon l'une quelconque des revendications 1 à 3, **caractérisé en ce que** les fluorophores sont choisis dans le groupe comprenant les xanthènes, les cyanines, les rhodamines, et les oxazines.

**5.** Procédé selon l'une quelconque des revendications 1 à 4, **caractérisé en ce qu'**une rhodamine de la formule générale suivante I

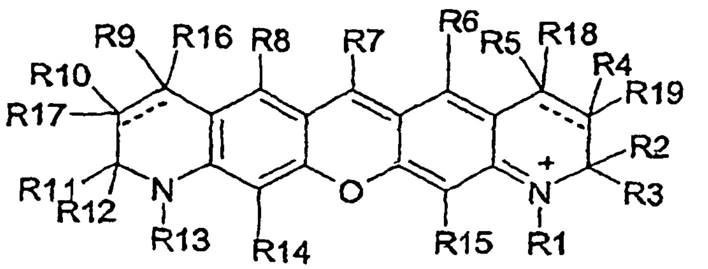

X-

(Formule I)

est employée en tant que fluorophore, dans laquelle R1 et R13 sont identiques ou différents et représentent : un atome d'hydrogène, un groupe alkyle ayant de 1 à 20 atomes de carbone, des unités polyoxyhydrocarbyle, un groupe phényle, un groupe phénylalkyle ayant de 1 à 3 atomes de carbone dans la chaîne alkyle, dans lesquels les radicaux alkyle et/ou phényle peuvent être substitués par un ou plusieurs groupes hydroxy, radicaux halogéno, groupes sulfo, carboxy ou alcoxycarbonyle, dans lesquels le groupe alcoxy peut posséder de 1 à 4 atomes de carbone;
R7 représente un groupe alkyle substitué par au moins un atome d'halogène, ayant de 1 à 20, de préférence de 1 à 7 atomes de carbone, ou un groupe phényle qui est substitué par un groupe carboxy ou alcoxycarbonyle se trouvant dans la position ortho, par rapport à l'atome de carbone lié au système pentacyclique et avec au moins un atome d'halogène, et dans lequel le groupe alcoxy peut posséder de 1 à 4 atomes de carbone, ou un groupe carboxyalkyle ou un groupe carboxyméthylèneoxy-alkyloxy;
les deux liaisons désignées par des traits interrompus, signifient que les deux atomes de carbone liés par la liaison en traits interrompus, peuvent être liés l'un à l'autre par une simple ou double liaison; R14, R15 et les autres positions non désignées par des symboles spécifiques du corps de base pentacyclique, pouvant être substituées par des groupes alkyle ayant 1 à 20 atomes de carbone; X- étant un contre-ion et au moins l'un des radicaux R1, R7 ou/et R13, étant couplé à une biomolécule.

**6.** Procédé selon la revendication 5, **caractérisé en ce que** le substituant R7 est un radical attracteur d'électrons.

**7.** Procédé selon l'une des revendications 1 à 4, **caractérisé en ce qu'**une oxazine de formule générale suivante II est employée en tant que fluorophore

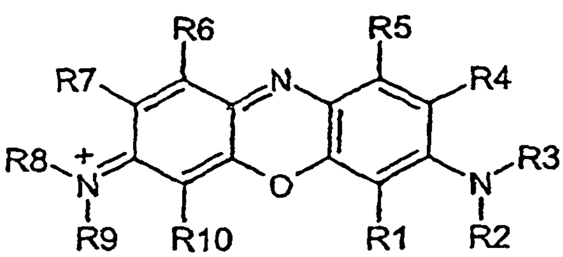

(Formule II)

dans laquelle R1, R4, R5, R6, R7, R10 représentent un atome d'hydrogène, un groupe alkyle, hydroxy, un atome d'halogène, un groupe carboxyle, sulfonyle ou amino, et
R2, R3 représentent un atome d'hydrogène, un groupe alkyle, alcoxy, des unités polyoxyhydroxycarbonyle, un groupe phényle, phénylalkyle, qui peuvent être substitués par un groupe hydroxy, sulfonyle, carboxy, amino, alcoxycarbonyle, R2 avec R1 ou R3 avec R4 pouvant former un pont en $C_4$ ou en $C_5$, saturé ou insaturé, et R8, R9 représentent un atome d'hydrogène, un groupe alkyle, alcoxy, des unités polyoxyhydroxycarbonyle, un groupe phényle phénylalkyle, qui peuvent être substitués par un groupe hydroxy, sulfonyle, carboxy, amino, alcoxycarbonyle, R2 avec R1 ou R3 avec R4 pouvant former un pont en $C_4$ ou $C_5$, saturé ou insaturé, et au moins l'un des radicaux R2, R3, R8 ou R9 représente un radical ne formant pas de pont, qui est couplé à une biomolécule, et au moins l'un des radicaux R2, R3, R8 et R9 représentant un radical formant un pont qui peut être substitué éventuellement par un groupe alkyle.

**8.** Procédé selon l'une quelconque des revendications précédentes, **caractérisé en ce que** le complexe chélate métallique est un complexe de ruthénium.

**9.** Procédé selon l'une quelconque des revendications 1 à 8, **caractérisé en ce que** la mesure de la fluorescence se réalise avec une résolution temporelle.

**10.** Procédé selon l'une quelconque des revendications 1 à 8, **caractérisé en ce que** la mesure de la fluorescence se réalise au moyen de la technique de modulation de phase.

**11.** Utilisation de complexes chélate métallique ayant des ions métalliques issus du groupe VII et VIII des éléments de transition, en tant que donneurs d'énergie de fluorescence-résonance, en combinaison avec des fluorophores de faible masse moléculaire, ayant une masse moléculaire comprise entre 300 d et 3 000 d dans un procédé pour détecter une interaction entre deux biomolécules dans un échantillon.

**12.** Utilisation selon la revendication 11, **caractérisée en ce que** le procédé est réalisé d'après le principe d'un test de liaison homogène.

**13.** Nécessaire de réactifs pour détecter un analyte dans un échantillon, **caractérisé en ce qu'**au moins une biomolécule marquée par un complexe chélate métallique ayant des ions métalliques issus du groupe VII et VIII des éléments de transition, ainsi qu'au moins une autre biomolécule qui est marquée par un fluorophore de faible masse moléculaire avec une masse moléculaire comprise entre 300 d et 3000 d, y sont contenues.

**14.** Combinaison de réactifs pour déterminer l'interaction entre les partenaires d'une paire de liaison à bio affinité, **caractérisée en ce qu'**un partenaire de la paire de liaison à bio affinité est marqué par un complexe chélate métallique ayant des ions métalliques issus du groupe VII et VIII des éléments de transition, et un autre partenaire de la paire de liaison à bio affinité est marqué par un fluorophore de faible masse moléculaire ayant une masse moléculaire comprise entre 300 d et 3000 d.

**15.** Procédé pour déterminer l'interaction entre des biomolécules marquées par un donneur, respectivement par un accepteur, basé sur le principe du transfert d'énergie de fluorescence-résonance et sur la mesure de la fluorescence résultante, **caractérisé en ce que** des complexes chélate métallique ayant des ions métalliques issus du groupe VII et VIII des éléments de transition sont mis en oeuvre, en tant que donneurs d'énergie, en combinaison avec des fluorophores de faible masse moléculaire avec une masse moléculaire comprise entre 300 d et 3000 d, en tant qu'accepteurs d'énergie.

Abbildung 1: Schema der Messvorrichtung

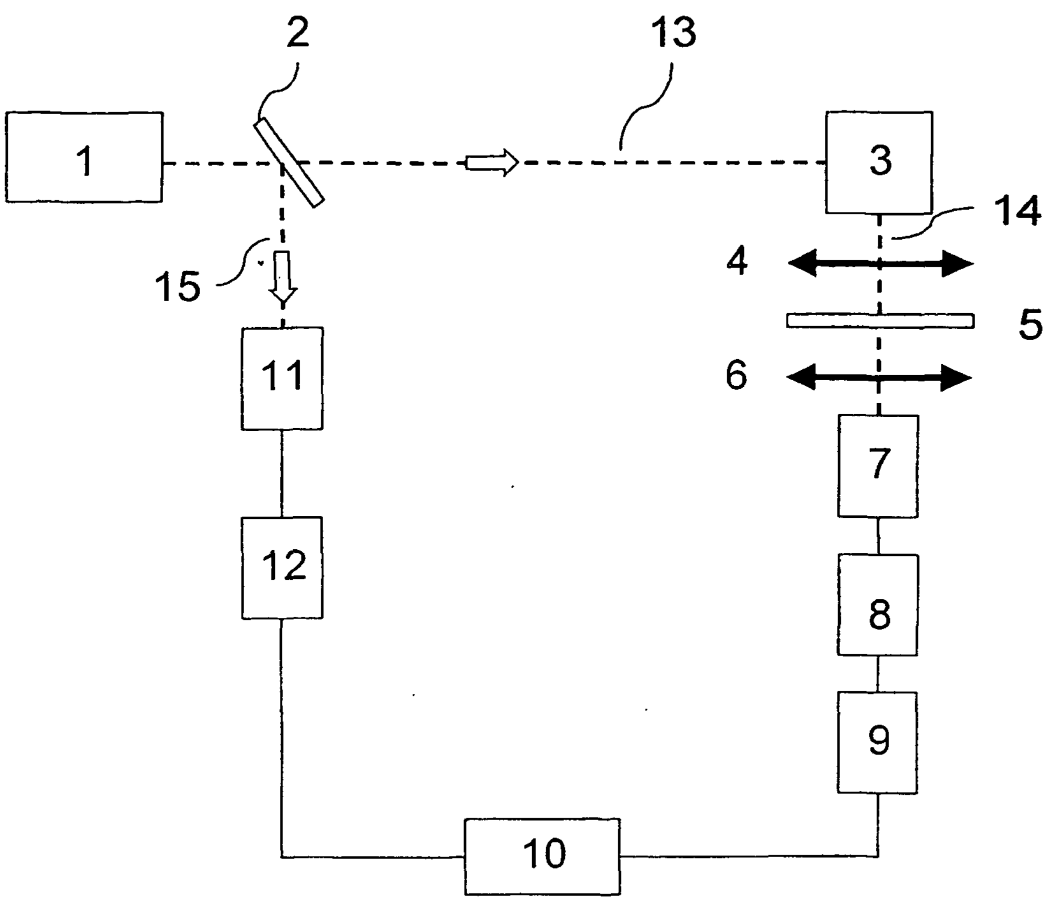


1. Lichtquelle
2. Quarzplatte
3. Probe
4. Sammellinse
5. Optische Filter
6. Fokussierlinse
7. Photomultiplier
8. Verstärker
9. Diskriminator
10. Zähler
11. Photodiode
12. Torschaltung
13. Anregungs-Lichtpuls
14. Fluoreszenzlicht
15. Lichtpuls zur Steuerung der Torschaltung (Trigger)

SEQUENCE LISTING

<110> Roche Diagnostics GmbH F. Hoffmann-La Roche AG

<120> Neue Farbstoffpaare für Fluoreszenz-Resonanz-Energie-Transfer-Messungen

<130> Case 19054 WO

<140>
<141>

<150> EP 00124995.2
<151> 2000-11-16

<160> 1

<170> PatentIn Ver. 2.1

<210> 1
<211> 9
<212> PRT
<213> Artificial Sequence

<220>
<223> Description of Artificial Sequence:HA-Peptid

<400> 1

```
Tyr Pro Tyr Asp Val Pro Asp Tyr Ala
  1               5
```